Europäisches Patentamt

(19) European Patent Office

Office européen des brevets

(11) Publication number: **0 172 619**

**A1**

(12) **EUROPEAN PATENT APPLICATION**

(21) Application number: 85304383.4

(22) Date of filing: 19.06.85

(51) Int. Cl.⁴: **C 12 N 15/00**
C 12 N 5/00, C 12 N 7/00
C 12 P 21/02, C 07 K 3/00
C 07 K 13/00

(30) Priority: 20.06.84 PC T/JP84/00323
02.05.85 PC T/JP85/00252

(43) Date of publication of application:
26.02.86 Bulletin 86/9

(84) Designated Contracting States:
CH DE FR GB LI

(71) Applicant: Takeda Chemical Industries, Ltd.
27, Doshomachi 2-chome Higashi-ku
Osaka-shi Osaka, 541(JP)

(72) Inventor: Sasada, Reiko
3-811, 48-3, Mibubojo-cho Nakagyo-ku
Kyoto-shi Kyoto 604(JP)

(72) Inventor: Onda, Haruo
21-16, Aza Junmatsu Tada-in
Kawanishi-shi Hyogo 666-01(JP)

(72) Inventor: Igarashi, Koichi
66-3, Shimogamo Miyazaki-cho Sakyo-ku
Kyoto-shi Kyoto 606(JP)

(74) Representative: Laredo, Jack Joseph et al,
Elkington and Fife High Holborn House 52/54 High
Holborn
London, WC1V 6SH(GB)

(54) Novel transformant and use thereof.

(57) This invention relates to animal cells transformed with a DNA having a region coding for a human IL-2 protein and, upstream therefrom, at least one promoter and to a method of producing such human IL-2 protein which comprises cultivating said transformants to thereby cause production and accumulation of said protein in the culture and recovering the same.

## Novel Transformant and Use Thereof

This invention relates to a novel transformant and a method of producing a human interleukin-2 protein.

Interleukin-2 [hereinafter referred to as IL-2; also called T-cell growth factor (TCGF)] is a lymphokine produced by T cells upon stimulation by a lectin or alloantigen [Science, 193, 1007-1008 (1976); Immunological Reviews, 51, 257-278 (1980)].          IL-2 enables long-period maintenance, by subculturing in vitro, of T cells by allowing their growth in vitro while maintaining their functions and, further, it is reported that IL-2 has an activity of promoting the mitogenic reaction of thymocytes (function as constimulator), restoring the ability of nude mouse spleen cells to produce antibodies to T-cell-dependent antigens (function as T cell replacing factor), or promoting the differentiation and proliferation of killer cells (function as killer helper factor) [The Journal of Immunology, 123, 2928-2929 (1979); Immunological Reviews, 51, 257-278 (1980)].

A large number of clones of killer T cells or helper T cells and, further, natural killer cells have so far been obtained through the utilization of IL-2 [e.g. Nature, 268, 154-156 (1977); The Journal of Immunology, 130, 981-987 (1983)]. In addition to such direct use in cloning T cells or natural killer cells, the use of IL-2 can result in selective in vitro growth of antigen-specific killer T cells capable of recognizing and destroying a certain particular antigen, for example a tumor antigen. By introducing into animals tumor-specific killer T cells grown in this manner, it is possible to control or inhibit tumor growth [The Journal of Immunology, 130, 1904-1909 (1980)]. Furthermore, it is known that IL-2 induces the production of interferon γ [The Journal of Immunology, 130, 1784-1789 (1983)] and that IL-2 activates natural killer cells [The Journal of Immunology, 130, 1970-1973 (1983)].

These experimental findings suggest the possible utility of IL-2 as an antitumor agent. It is further known that IL-2 restores the helper T cell function in nude mice which are deficient in thymus functions [European Journal of Immunology, 10, 719-722 (1980)] and restores the induction of killer T cells against allogenic cells [Nature, 284, 278-280 (1980)], and therefore IL-2 can be expected to be useful in the treatment of immunocompromised patients.

Taniguchi et al. [Nature, 302, 305-310 (1983)] and Devos et al. [Nucleic Acids Research, 11, 4307-4323 (1983)] separately cloned the human IL-2 gene, described the amino acid sequence of the human IL-2 polypeptide as estimated therefrom and reportedly succeeded in causing the expression of said gene.

In the work of Devos et al. cited above, said gene was expressed in Escherichia coli, and the protein obtained in that manner is estimated to be a unglycosylated protein.

On the other hand, the report of Taniguchi et al. describes that the human IL-2 gene was expressed using the promoter of SV-40 and transfecting simian COS-7 cells. This method estimatedly can produce glycosylated human IL-2. However, the cells used there cannot be maintained under this condition for a long term but have only temporary protein-producing activity. Therefore, the method is not a satisfactory one from the practical viewpoint.

The present inventors established an industrially-advantageous method of producing a glycosylated human IL-2 protein by producing an animal cell transformant having a DNA coding for the human IL-2 protein and cultivating said transformant and thus completed the present invention.

The present invention provides an animal cell transformed with a DNA having a region coding for human IL-2 protein and at least one promoter upstream therefrom as well as a method of producing a human IL-2 protein which comprises cultivating said transformant to thereby cause production and accumulation of a human IL-2 protein and recovering the same.

The DNA having a human IL-2 protein-encoding region, which is to be used in the practice of the invention, may be any of those DNAs which code for the human IL-2 protein having the formula

Ala Pro Thr Ser Ser Ser Thr Lys Lys Thr Gln Leu

Gln Leu Glu His Leu Leu Leu Asp Leu Gln Met Ile

Leu Asn Gly Ile Asn Asn Tyr Lys Asn Pro Lys Leu

Thr Arg Met Leu Thr Phe Lys Phe Tyr Met Pro Lys

Lys Ala Thr Glu Leu Lys His Leu Gln Cys Leu Glu

Glu Glu Leu Lys Pro Leu Glu Glu Val Leu Asn Leu

Ala Gln Ser Lys Asn Phe His Leu Arg Pro Arg Asp

Leu Ile Ser Asn Ile Asn Val Ile Val Leu Glu Leu

Lys Gly Ser Glu Thr Thr Phe Met Cys Glu Tyr Ala

Asp Glu Thr Ala Thr Ile Val Glu Phe Leu Asn Arg

Trp Ile Thr Phe Cys Gln Ser Ile Ile Ser Thr Leu

Thr

or a polypeptide substantially equivalent thereto in activity.

The human IL-2 structural genes described in the reports of

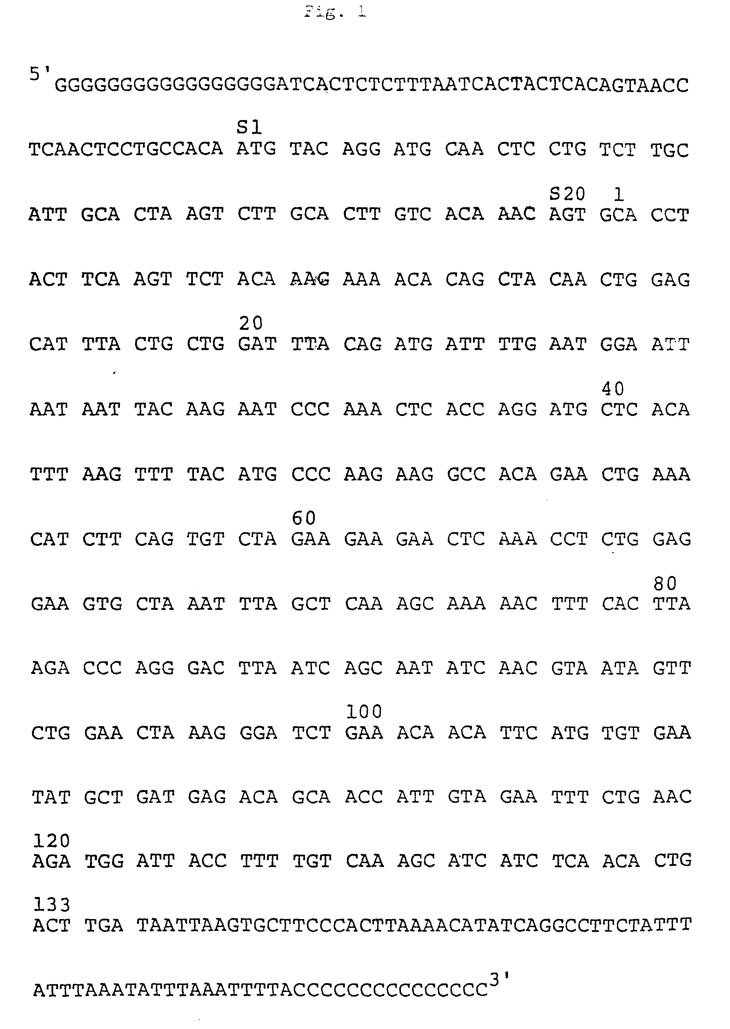

Taniguchi et al. and Devos et al. and the DNA lacking four codons at the 5'terminus of the human IL-2 structural gene described in Japanese Patent Application No. 235638/1983 (filed December 13, 1983) are examples. A particularly preferred one is, for example, the DNA having the base sequence represented by the codons 1 to 133 shown in Fig. 1.

As the promoter, there may be used any of the promoters for the expression in animal cells.

The above-mentioned DNA may have at least one enhancer in addition to a promoter.

The promoter and enhancer are preferably animal virus-derived ones, such as derived from simian virus, murine retrovirus or human retrovirus.

More specifically, there may be mentioned, as the promoter, the promoter region of SV (simian virus) 40 [Okayama et al., Molecular and Cellular Biology, 3, 280-289 (1983)] and the promoter occurring in various retrovirus LTR (long terminal repeat) regions.

As the retrovirus LTR region-derived promoters, there may be mentioned, for example, the following:

o Abelson murine leukemia virus (A-MuLV) [Goff, S. P. et al., Cell, 22, 777-785 (1980)],

o Moloney murine leukemia virus (M-MuLV) [Niwa et al., Cell, 32. 1105-1113 (1983)],

o Adult T cell leukemia virus (ATLV) [Yoshida et al., Proceedings of the National Academy of Sciences of USA, 79, 6899-6902 (1982)], and

o Avian sarcoma virus (ASV) [Kitamura et al., Nature, 297, 205-208 (1982)].

In the practice of the invention one or two or more of the above-mentioned promoters may be used.

As the enhancer, there may be mentioned, for example the enhancer occuring in the SV 40 promoter region [Okayama et al., vide supra] and the enhancer occurring in a retrovirus LTR region, and particularly preferred is the enhancer in the repeating base sequence portion of the above-mentioned LTR regions.

As the virus-derived enhancer, there may be mentioned, for example, the following:

A-MuLV [Goff, S. P. et al., vide supra],

M-MuLV [Niwa et al., vide supra],

ATLV [Yoshida et al., vide supra] and

ASV [Kitamura et al., vide supra].

In the practice of the invention, one or two or more of the enhancers such as mentioned above may be used.

The above-mentioned promoter and enhancer can be selected for their proper use depending on the animal cell species to be transformed.

For transforming murine cells or hamster cells, for instance, the promoter and enhancer in the LTR region of a retrovirus, particularly A-MuLV or M-MuLV and/or the promoter and enhancer in the SV 40 promoter region are preferable.

For transforming human cells, the promoter and enhancer in the LTR region of a retrovirus, preferably a human-derived retrovirus, in particular ATLV, are preferred.

The gene expression system as disclosed herein by way of example, which comprises both the promoter (optionally with the enhancer therefor) of a retrovirus and the SV40 promoter (optionally with the enhancer therefor), may lead to efficient expression of a particular gene in a variety of animal cells upon transformation of animal cells, followed by collecting animal cell transformants (optionally following cloning) and allowing the production of the gene expression product.

Therefore, the above expression system may be used advantageously in the expression, in animal cells, of not only the IL-2 gene but also genes for useful proteins such as lymphokines (e.g. interferon-α, interferon-β, interferon-γ, lymphotoxin, tumor necrosis

factor) and hormones (e.g. insulin, somatostatin, human growth hormone).

For producing a particular protein at high efficiency in animal cells, a procedure for increasing the copy number of a particular gene (e.g. IL-2 gene) per cell may also be followed in addition to the elaboration and use of promoter and enhancer. For instance, a gene which is amplifiable and may also be used as a selective marker as desired, such as the dihydrofolate reductase (DHFR) gene, can be inserted into the plasmid at a site close to the gene to be expressed (U. S. Patent No. 4,399,216).

The DNA for animal cell transformation, which is to be used in the practice of the invention and has a region coding for a human IL-2 protein and a promoter upstream therefrom, can be produced, for example, from a cloned plasmid containing a DNA (cDNA) having a region coding for IL-2 protein, a plasmid containing a promoter, for example a virus-derived promoter, (further an enhancer as necessary) and a splice region and a plasmid containing, for example a poly(A) addition region.

For illustrating the method of producing the above-mentioned DNA for animal cell transformation more specifically, a case is described below in which the DNA shown in Fig. 1 (cDNA in pILOT135-8) is used as the DNA having a region coding for human IL-2 protein.

pILOT135-8 is cleaved with the restriction enzymes PstI and StuI, followed by ligation with the BamHI linker at the StuI site. Separately, a plamid having a promoter and a splice region [e.g. pP11; Okayama et al., vide supra] is cleaved with HindIII and PstI, and a plasmid having a poly(A) addition region [e.g. pCDV1; ibid.] with HindIII and BamHI. The three DNA fragments are ligated together and the product is used for the transformation of Escherichia coli DH1, for instance. Ampicillin-resistant colonies are selected, cultured and extracted, whereby a plasmid (I) for animal cell transformation which has a region coding for a human IL-2 protein can be isolated. This plasmid (I) has a region coding for human IL-2 between the promoter/splice region of the SV40 DNA and the poly(A) addition region.

If necessary, the above plasmid (I) may be cleaved at the HindIII site occurring upstream from the SV40 promoter region, a base sequence repeat portion DNA fragment of the cloned LTR region of the above-mentioned retrovirus isolated, purified and ligated with the HindIII linker, and the ligation product inserted into the above-mentioned plasmid (I) at the HindIII site thereof, whereby a plasmid (II) for animal cell transformation can be constructed. This plasmid (II) has the retrovirus-derived promoter and enhancer and, downstream therefrom, a region coding for human IL-2 protein between the promoter/splice region of the SV40 DNA and the poly(A) addition region.

If necessary, the above plasmid (II) is further cleaved, for example with the restriction enzyme ClaI and, after inactivation of ClaI, a ClaI DNA fragment upstream from the LTR region is removed by the action of T4 DNA ligase, whereby a plasmid (III) with the HindIII cleavage site only between the LTR region and the SV40 promoter region is constructed. The plasmid (III) is cleaved with the restriction enzymes HindIII and XhoI, both cohesive ends are rendered blunt and ligated together by the T4 DNA ligase reaction, whereby a plasmid (IV) for animal cell transformation may be constructed. The plasmid (IV) has the promoter and enhancer of the retrovirus LTR region and, downstream therefrom, a human IL-2 protein-encoding region.

If desired, plasmid (IV) is cleaved with the restriction enzyme ClaI, the cohesive ends are rendered blunt and then a HindIII linker is joined thereto. The product is cleaved with HindIII, deprived of the above-mentioned retrovirus (Mu-LV)-derived promoter and enhancer are removed, and the plasmid is made circular. From the ATLV cDNA (Yoshida et al., vide supra), the LTR region thereof is excised and inserted into the above plasmid to give a plasmid (V) having the promoter and enhancer from the human retrovirus LTR region and, downstream therefrom, the human IL-2 gene.

Separately, the HindIII cleavage site of plasmid (IV) is converted to a XhoI site, and the PstI cleavage site on the 5' end side of the IL-2 gene to an EcoRI site. A BglII cleavage site is inserted into said plasmid directly before the BamHI cleavage site on the 3' end side and a ClaI cleavage site and a HindIII cleavage site are inserted thereinto downstream from the poly(A) addition region, whereby plasmid (VI) is obtained.

The plasmid (VI) is digested with ClaI and a DNA fragment thereof containing MuLV-LTR, IL-2 gene, and so on is inserted into a hamster DHFR gene cDNA-containing plasmid at the ClaI cleavage site. This gives a plasmid (VII) having the IL-2 gene downstream from the retrovirus-derived promoter and, further downstream therefrom, the SV40 promoter and DHFR gene.

Furthermore, a BamHI DNA fragment of a DHFR gene-containing cDNA is inserted into the plasmid (VI) at the BglII cleavage site to give a plasmid (VIII) having the IL-2 gene and DHFR gene downstream from the retrovirus-derived promoter.

The animal cell transformants according to the invention may be produced, for example, by transforming animal cells with a DNA (plasmid) having a human IL-2 protein-encoding region and, upstream therefrom, a promoter, followed if necessary by selection and isolation.

As the above-mentioned animal cells, there may be used any animal cells which allow the expression of the IL-2 gene, for instance mouse cells [e.g. L cells; Proceedings of the Society of Experimental Biology and Medicine, 92, 893 (1956)], rat cells [e.g. NRK cells; Journal of Cell Physiology, 94, 335 (1978)], chicken- or duck-derived cells, human cells [e.g. FL cells; Proceedings of the Society of Experimental Biology and Medicine, 94, 532 (1957)] and hamster cells [e.g. CHO cells; Proceedings of the National Academy of Sciences of USA, 77, 4216 (1980)].

The transformation is carried out by the contrans-
formation method [Wigler et al., Cell, 16, 777-785 (1979)],
whereby the desired animal cell transformants may advantageously be selected and isolated.

Thus, cells of an animal deficient in a specific gene
[thymidine kinase (TK) gene, adenine phosphoribosyl trans-
ferase gene, DHRF gene, etc.], for example mouse TK gene-dificient
L cells or hamster DHRF gene-deficient CHO cells, are transformed using simultaneously a DNA (plasmid) having
the above-mentioned human IL-2 protein-encoding region and
so forth and, as a marker, a plasmid having the above gene
deficient in said cells, or normal cells are cotransformed
using simultaneously, as a marker, a plasmid containing an
antibiotic (e.g. neomycin) resistance gene.  In the former
case, the desired transformants may easily be selected and
isolated by the known method [Wigler et al., vide supra;
Lee et al., Nature, 294, 228-232(1981)] and, in the latter,
by adding the corresponding antibiotic or a derivative
thereof [e.g. geneticin (G418)] to the cell cutlture
medium, followed by cultivation [Colbère-Garapin et al.,
Jornal of Molecular Biology, 150, 1-14 (1981).

The former method and latter method may also be used
in combination for transformant selection and collection.

The cloned animal cell transformants having the IL-2
gene have been provided for the first time by cotransforma-
tion and may be used advantageously in the production of
glycosylated IL-2 and so forth.

The human IL-2 protein of this invention can be produced by cultivating the above-mentioned transformants according to the invention to thereby cause production and accumulation of said protein in the culture and recovering said protein.

The cultivation is conducted using a medium for animal cell culture, such as MEM medium supplemented with bovine fetal serum or a mammalian serum-derived composition by subjecting a mammalian serum to a contaminant microorganism inactivating step and a purification step involving salting out and desalting   [Japanese Patent Application No. 521/1984 (filed January 9, 1984)].

The cell culture is generally conducted at 30-40°C for 2-10 days.

When a transformant carrying a gene amplification gene (e.g. DHFR gene) is used, the yield of a particular protein can be increased by first cultivating the transformant under appropriate gene amplification conditions (for the DHFR gene, in the presence of methotrexate, generally in a concentration of 1-100 μM) to thereby cause marker gene amplification, which results in an increase in the copy number of a particular gene.

The IL-2 protein produced and accumulated in the culture may be separated from the culture supernatant and purified by an appropriate combination of known separation and purification methods, although the culture broth after removal of cells may be concentrated and dried and the dried product may also be used as it is.

Such known separation and purification methods include, among others, a method taking advantage of solubility difference, such as salting out or precipitation by adding a solvent; a method principally taking advantage of molecular weight difference, such as dialysis, ultrafiltration, gel filtration or SDS-polyacrylamide gel electrophoresis; a method utilizing electric charge difference, such as ion exchange chromatography; a method making use of specific affinity, such as affinity chromatography; a method making good of hydrophobicity difference, such as reversed-phase high-performance liquid chromatography; and a method utilizing difference in isoelectric point, such as isoelectric focusing.

If necessary, the IL-2 protein-containing solution obtained here may be converted to the powder form by lyophilization. In this lyophilization, there may be added a stabilizer such as sorbitol, mannitol, dextrose, maltose, glycerol or human serum albumin (HSA).

By using the method of producing a human IL-2 protein using the animal cell transformants according to the invention, a glycosylated human IL-2 protein may be produced easily and in large quantitites.

The glycosylated human IL-2 protein obtained in accordance with the invention has low toxicity and substantial-

- 14 -

0172619

ly the same activities as known natural human IL-2. The phrase "substantially the same activities as natural human IL-2" as used herein means the following biological and immunological activities, among others. Thus, it has an activity to enable normal T cells or natural killer cells to proliferate while maintaining their functions. Therefore, the human IL-2 protein according to the invention may be used in growing and subculturing T cells or natural killer cells in vitro for a long period of time or in cloning them. This property may also be utilized in human IL-2 activity assay.

Furthermore, the human IL-2 protein according to the invention may enable the in vitro selective growth of antigen-specific killer T cells capable of recognizing and destroying a tumor antigen, for instance, or of natural killer cells capable of killing a tumor irrespective of experience or nonexperience of antigenic sensitization. When said killer T cells are introduced into a living body, simultaneous inoculation with the human IL-2 protein according to the invention increases the antitumor effect. Therefore, said protein may be used in the prevention and treatment of tumors in warm-blooded animals (e.g. mouse, rat, rabbit, dog, cat, pig, horse, sheep, cattle, human) or in the treatment of immunocompromised state in such animals.

For the purpose of enabling proliferation of T cells in vitro, the human IL-2 protein according to the invention may be added to the medium in a concentration of about 0.01

- 15 -

0172619

to 1 unit/ml, preferably about 0.1 to 0.5 unit/ml.

In an embodiment in which it is used for the purpose of enabling proliferation of T cells in vitro, the human IL-2 protein according to the invention is added, for example, in a concentration of 0.1-0.5 unit/ml, to a cell suspension containing alloantigen-sensitized T cells as obtained by 3-day mixed lymphocyte culture in RPMI 1640 medium containing 20% fetal bovine serum at 37°C and in the presence of 5% $CO_2$ of T cells (1 x $10^6$ cells/ml) separated from human peripheral blood and B cell transformants (1 x $10^6$ cells/ml) irradiated with X-ray (1,500 rad), and the culture of said alloantigen-sensitized T cells is continued for about 1 month while exchanging the medium at about 1-week intervals.

In using the human IL-2 protein according to the invention as a prophylactic and therapeutic agent against tumors, said protein may be mixed with a per se known carrier for dilution and administered parenterally or orally in the form of injection or capsules, for instance. It can be used either alone or in combination with killer T cells or natural killer cells grown in vitro as mentioned above.

The human IL-2 protein according to the invention is a glycosylated one and has substantially the same biological activities as known human IL-2 separated from nature. Therefore, it may be used in the same manner as said natural one.

As compared with human IL-2 produced in Escherichia coli, for instance, by the recombinant DNA technology, the

- 16 -

0172619

human IL-2 protein according to the invention, which is a glycosylated one, is higher in solubility in water and may be purified and made up into pharmaceutical preparations with advantage and may be used with advantage.

The human IL-2 activity data given in this specification were obtained in the following manner.

Thus, a transformant culture supernatant or an IL-2-containing sample was added to a medium containing, in the form of a suspension, a mouse cell line growing in the IL-2 concentration-dependent manner and, after incubation, the proliferation of said cell line was determined with the tritiated thymidine uptake as an index. For the calculation of the number of units (U) for the sample, a standard IL-2 sample (1 U/ml) was used always in parallel for the assay, and said number of units was calculated from the ratio therebetween.

More specifically, an IL-2-dependent mouse cell line [(NKC3), Hinuma et al., Biochemical and Biophysical Research Communications, 109, 363-369 (1982)] maintained by passage in 20% FCS-added RPMI 1640 medium containing conditioned medium and the transformant cell culture supernatant or human IL-2 at 37°C in the presence of 5% $CO_2$ is washed twice with serum-free RPMI 1640 medium and resuspended in 20% FCS-added RPMI 1640 medium in a concentration of 6 x $10^5$ cells/ml.

A sample containing the transformant cell culture supernatant or IL-2 is distributed in 50-$\mu$l portions into the first-row wells of a 96-well microtiter plate (Nunc, Denmark) and a doubling dilution series is made up to the 12th row by using 50-µl portions of 20% FCS-added RPMI 1640 medium. Thereafter, the above-mentioned NKC3 cell suspension is distributed in 50-µl portions into the wells. Incubation is conducted at 37°C in the presence of 5% $CO_2$ for 24 hours. At hour 20 of incubation, 1 µCi of tritiated thymidine (Amersham, Great Britain) is added to each well. After 4 further hours of incubation, cells are recovered on a glass filter using a cell harvester (Flow, USA) and measured for the tritiated thymidine uptake using a liquid scintillation counter. In assaying, the same procedure as above is followed with a standard IL-2 sample for measuring the tritiated thymidine uptake.

The unit (U) number calculation is carried out by the probit conversion method according to Journal of Immunology, 120, 2027-2032 (1978). Thus, a standard IL-2 sample (the centrifugal supernatant of a culture of human peripheral blood lymphocytes suspended in 10% FCS-added RPMI 1640 medium in a concentration of $5 \times 10^6$ cells/ml and incubated at 37°C in the presence of 5% $CO_2$ with 40 µg/ml of concanavalin A and 15 ng/ml of 12-O-tetradecanoylphorbol-13-acetate added being defined as having an activity of 1 U/ml) is serially diluted and the maximum uptake value in the dilution series is regarded as 100% and used for the calculation of the relative uptake value (%) for each dilution step. The numerical values obtained are plotted on normal probability paper and the dilution factor to which an uptake of 50% corresponds is determined graphically. In the same manner, the dilution factor corresponding to an uptake of 50% is determined for the IL-2-containing sample to be assayed.

The IL-2 activity (U/ml) for a sample is calculated according to the formula:

$$\frac{\text{Dilution factor at which sample shows 50\% uptake}}{\text{Dilution factor at which standard IL-2 shows 50\% uptake}}$$

The specific activity of natural IL-2 obtained from human peripheral blood was 20,000-70,000 U/mg as determined by the above assay method.

In the present specification and drawings, the bases and so on, when indicated by abbreviations, are indicated by abbreviations according to the IUPAC-IUB Commission on Biochemical Nomenclature or by abbreviations conventional in the relevant field. The following is a list of examples:

DNA   : Deoxyribonucleic acid

cDNA  : Complementary deoxyribonucleic acid

A     : Adenine

T     : Thymine

G     : Guanine

C     : Cytocine

RNA   : Ribonucleic acid

mRNA  : Messenger ribonucleic acid

dATP  : Deoxyadenosine triphosphate

dTTP  : Deoxythymidine triphosphate

dGTP  : Deoxyguanosine triphosphate

dCTP  : Deoxycytidine triphosphate

ATP   : Adenosine triphosphate

EDTA  : Ethylenediaminetetraacetic acid

## BRIEF DESCRIPTION OF DRAWINGS

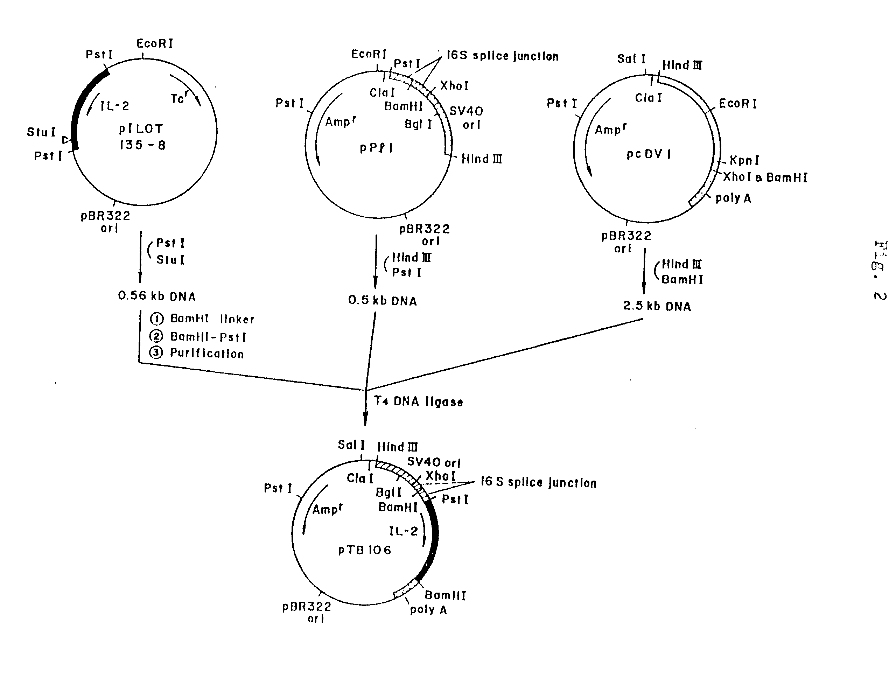

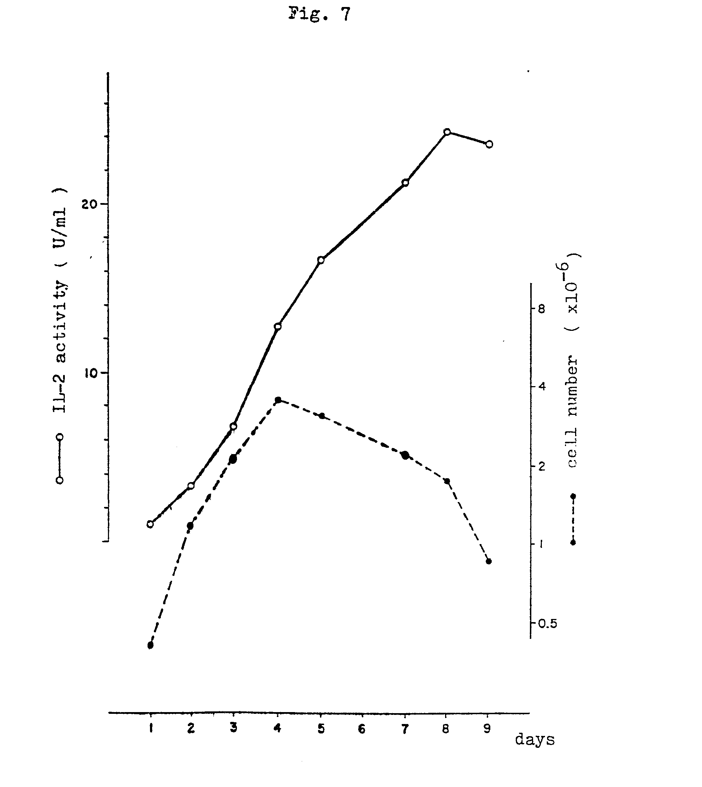

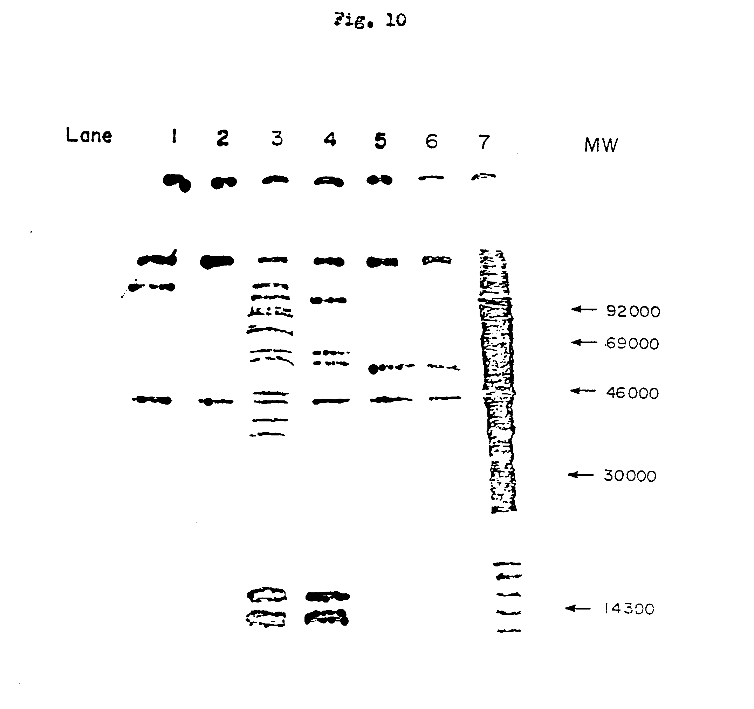

Fig. 1 shows the primary structure (base sequence) of the IL-2 gene of the plasmid pILOT135-8 obtained in Reference Example (vii), and Fig. 2, Fig. 3, Fig. 4, Fig. 5 and Fig. 6 show the construction scheme for the plasmid pTB106 for animal cell transformation in Example 1 (i), for the plasmids pTB213 and pTB215 in Example 1 (ii), for the plasmid pTB314 in Example 1 (iii), pTB385 in Example 1 (iv), and pTB485, and pTB487 in Example (v), respectively. Fig. 7, Fig. 8 and Fig. 9 show the number of transformant cells during cultivation in Example 2 and the results of IL-2 activity assay of the culture supernatant. Fig.10 shows the results of autoradiography in Example 3, lanes 1 and 2 being for the reaction of normal antiserum with 10-fold and 100-fold dilutions, respectively and lanes 3, 4, 5, and 6   for the reaction of anti-human IL-2 antiserum with 10-fold, 100-fold, 1,000-fold and 10,000-fold dilutions, respectively.  Lane 7 shows the crude lysate prepared from cells labelled with $^{35}$S-methionine.

## Examples

The present invention will be explained more concretely by the following Reference Examples and Examples, but the present invention is not rectricted by them.

The transformants disclosed in the Examples are deposited with the          Institute for Fermentation, Osaka (IFO) under the following deposit numbers, respectively.

Mouse L-IL213-3 cell:  IFO-50049

Human FL-IL385-6 cell:  IFO-50050

Hamster C-IL485-14 cell:  IFO-50051

The transformant Escherichia coli DH1/pTF4 is deposited with IFO under the deposit number IFO-14299, and with the Fermentation Research Institute, Agency of Industrial Science and Technology, Ministry of International Trade and Industry, Japan (FRI) under the accession number FERM BP-628.

Reference Example    Isolation of human IL-2 gene-containing plasmid

(i)  Isolation of mRNA coding for human IL-2

For inducing IL-2, lymphocytes prepared from human peripheral blood were incubated at 37°C in RPMI 1640 medium (containing 10% fetal bovine serum) containing 12-O-tetradecanoylphorbol-13-acetate (TPA) (15 ng/ml) and concanavalin A (40 µg/ml). After 24 hours, 1 x $10^{10}$ lymphocyes thus induced were destroyed and denatured in a solution containing 5 M guanidine thiocyanate, 5% mercaptoethanol, 50 mM Tris·HCl pH 7.6, and 10 mM EDTA with a Teflon homogenizer. Then, sodium N-lauroylsarcosinate was added in a concentration of 4% and the mixture, after homogenization, was layered on 7.5 M cesium chloride solution (7.5 M cesium chloride, 0.1 M EDTA), followed by centrifugation at 15°C and 24,000 rpm for 48 hours using a Beckman SW28 rotor, whereby an RNA precipitate was obtained. This RNA precipitate was dissolved in 0.25% sodium N-lauroylsarcosinate solution and thereafter precipitated with ethanol to give 10 mg of RNA. This RNA was allowed to be adsorbed on an oligo(dT)-cellulose column in a high-concentration salt solution [0.5 M NaCl, 10 mM Tris·HCl pH 7.6, 1 mM EDTA, 0.3% SDS] and the poly(A)-containing mRNA was eluted with a low-concentration salt solution (10 mM Tris·HCl pH 7.6, 1 mM EDTA, 0.3% SDS), whereby 300 µg of poly(A)-containing mRNA was isolated.

This mRNA was further precipitated with ethanol, the precipitate was dissolved in 0.2 ml of a solution (10 mM Tris·HCl pH 7.6, 2 mM EDTA, 0.3% SDS) and, after treatment at 65°C for 2 minutes, fractionated by 10-35% sucrose density gradient centrifugation (centrifugation at 20°C and 25,000 rpm for 21 hours using a Beckman SW28 rotor) to give 22 fractions. Aliquots of each fraction were injected into oocytes of Xenopus laevis and the IL-2 activity in the protein synthesized was assayed. The IL-2 activity was found with fractions 11-15 (sedimentation coefficients 8S-15S). The IL-2 mRNA in these fractions weighed about 25 µg.

(ii) Synthesis of single-stranded DNA

Using the mRNA obtained in the above and reverse transcriptase, incubation was conducted in 100 µl of a reaction mixture (5 µg of mRNA, 50 g of oligo(dT), 100 units of reverse transcriptase, 1 mM each of dATP, dCTP, dGTP and dTTP, 8 mM $MgCl_2$, 50 mM KCl, 10 mM dithiothreitol, 50 mM Tris·HCl pH 8.3) at 42°C for 1 hour, deproteinization was then effected with phenol, and RNA was decomposed and removed by treatment with 0.1 N NaOH at 70°C for 20 minutes.

(iii) Synthesis of double-stranded DNA

The single-stranded complementary DNA thus synthesized was incubated in 50 µl of a reaction mixture [the same reaction mixture as above except that it contained neither mRNA nor oligo(dT)] at 42°C for 2 hours, whereby a double-stranded DNA was synthesized.

(iv) Addition of dC tail

This double-stranded DNA was subjected to the action of nuclease S1 in 50 µl of a reaction mixture (double-stranded DNA, 0.1 M sodium acetate pH 4.5, 0.25 M NaCl, 1.5 mM $ZnSO_4$, 60 units of S1 nuclease) at room temperature for 30 minutes, followed by deproteinization with phenol and DNA precipitation with ethanol. The precipitate was subjected to the action of terminal transferase in 50 µl of a reaction mixture (double stranded DNA, 0.14 M potassium cacodylate, 0.3 M Tris (base) pH 7.6, 2 mM dithiothreitol, 1 mM $CoCl_2$, 0.15 mM dCTP, 30 units of terminal transferase) at 37°C for 3 minutes, whereby the double-stranded DNA was extended by a chain of about 15 deoxycytidines at each 3' end thereof. This series of reactions gave about 300 ng of a double-stranded DNA having deoxycytidine chains.

(v)  Cleavage of Escherichia coli plasmid and addition of dG tail

Separately, 10 μg of the Escherichia coli plasmid pBR322 DNA was subjected to the action of the restriction enzyme PstI in 50 μl of a reaction mixture (10 μg of DNA, 50 mM NaCl, 6 mM Tris·HCl pH 7.4, 6 mM MgCl$_2$, 6 mM 2-mercaptoethanol, 100 μg/ml of bovine serum albumin, 20 units of PstI) at 37°C for 3 hours to thereby cleave the one PstI recognition site occuring in the pBR322 DNA, followed by deproteinization with phenol. The cleavage product was subjected to the action of terminal transferase in 50 μl of a reaction mixture (10 μg of DNA, 0.14 M potassium cacodylate, 0.3 M Tris (base) pH 7.6, 2 mM dithiothreitol, 1 mM CoCl$_2$, 0.15 mM GTP, 30 units of terminal transferase) at 37°C for 3 minutes to thereby extend the above plasmid pBR322 DNA by a chain of about 17 deoxyguanidines at each 3' end thereof.

(vi)  Annealing of cDNA and transformation of Escherichia coli

The thus-obtained synthetic double-stranded DNA (0.1 μg) and 0.5 μg of the above-mentioned plasmid pBR322 were annealed by heating in a solution comprising 0.1 M NaCl, 50 mM Tris·HCl pH 7.6 and 1 mM EDTA at 65°C for 2 minutes and then at 45°C for 2 hours, followed by slow cooling. The product was used for transformation of Escherichia coli by the method of Enea et al. [Journal of Molecular Biology,

- 24 -

0172619

96. 495-509 (1975)].

(vii)   Isolation of cDNA-containing plasmid

In this way, about 20,000 tetracycline-resistant strains were isolated.  These DNAs were each immobilized on a nitrocellulose filter.  Based on the amino acid sequence of IL-2 as reported by Taniguchi et al. [vide supra], the base sequences ($^{5'}$AAA CAT CTT CAG TGT$^{3'}$ and $^{5'}$ACA TTC ATG TGT GAA$^{3'}$) corresponding to amino acids Nos. 74-78 (Lys$^{74}$-His-Leu-Gln-Cys) and amino acids Nos. 122-126 (Thr$^{122}$-Phe-Met-Cys-Glu) were chemically synthesized by the phosphotriester method [Crea et al., Proceedings of the National Academy of Sciences, USA, 75, 5765-5769 (1978)].

These oligonucleotides were labeled with $^{32}$P at the 5' end by conducting the reaction, using T4 polynucleotide kinase, in 50 µl of a reaction mixture (0.20 µg of oligonucleotide, 50 mM Tris·HCl pH 8.0, 10 mM MgCl$_2$, 10 mM mercaptoethanol, 50 µCi of γ-$^{32}$P ATP, 3 units of T4 polynucleotide kinase).  These labeled oligonucleotides, as probes, were annealed with the above-mentioned DNA fixed on the nitrocellulose filter, by the method of Lawn et al. [Nucleic Acids Research, 9, 6103-6114 (1981)] and 4 strains reacting with the above two oligonucleotide probes were isolated.  The plasmid DNA was isolated from each of these strains by the alkaline method [Birnboim et al., Nucleic Acids Research, 7, 1513-1524 (1979)].  The insert of the plasmid DNA was excised using the restriction enzyme PstI.  Of the plasmids isolated, the one which contained the longest insert fragment was selected and named pILOT135-8.

The primary structure (base sequence) of the cDNA sequence inserted in this plasmid pILOT135-8 was determined by the dideoxynucloetide method and by the Maxam-Glibert method. The primary structure is given in Fig. 1.

Example 1  Production of animal cell transformant

(i)  Construction of plasmid pTB106

The IL-2 gene portion (nucleotides Nos. 1-559) was excised from the plasmid pILOT135-8 (obtained in Reference Example) using the restriction enzymes PstI and StuI, followed by agarose electrophoresis, which gave a DNA fragment (0.56 kb). A 0.5-µg portion of this DNA was dissolved in 15 µl of ligation buffer (66 mM Tris·HCl pH 7.6, 6.6 mM MgCl$_2$, 10 mM DTT, 66 µM ATP), followed by addition of 0.2 µg of BamHI linker (CCGGATTCCGG) phosphorylated at the 5' end and 2 units of T4 DNA ligase. After 17 hours of reaction at 14°C, the ligase was inactivated by heat treatment at 65°C for 10 minutes. Then, 5 volumes of distilled water was added and the dilution was treated further with 30 units of BamHI and 8 units of PstI in BamHI buffer (10 mM Tris·HCl pH 8, 100 mM NaCl, 10 mM MgCl$_2$, 1 mM mercaptoethanol, 100 µg/ml bovine serum albumin) for 3 hours. Using a Sepharose 4B column (0.5 cm in diameter, 15 cm long), the linker fraction and the linker-joined IL-2 DNA were separated from each other and the BamHI linker-joined IL-2 DNA (the other end being PstI) was recovered by precipitation with ethanol.

The IL-2 gene portion (nucleotides Nos. 1-559) mentioned above can be also obtained by ligation of chemically synthesized nucleotides coding for IL-2 signal peptide sequence (codons Nos. S1 to S20 in Fig. 1) and DNA fragment excised from the plasmid pTF4 in transformant Escherichia coli DH1/pTF4 using restriction enzymes EcoRI and PstI followed by digestion with S1 nuclease.

Separately, the known plasmid pPl1 [Okayama et al., vide supra] was cleaved with the restriction enzymes HindIII and PstI, and a 0.5 kb DNA having the SV40 promoter and a splice region was separated by agarose electrophoresis. Furthermore, the plasmid pcDV1 [Okayama et al., vide supra] was cleaved with the restriction enzymes BamHI and HindIII, and a 2.5 kb DNA fragment containing the poly(A) addition region of SV40 and the replication origin and ampicillin resistance gene derived from the plasmid pBR322 was produced in a similar manner.  These three DNA fragments were joined together using T4 DNA ligase and the product was used for transforming Escherichia coli DH1 [Maniatis et al., Molecular Cloning, pages 249-255, Cold Spring Harbor Laboratory (1982)]. The ampicillin-resistant colonies obtained were screened for plasmid isolation by the Birnboim-Doly method and the construction of a plasmid for animal cell transformation, pTB106, containing the human IL-2 gene was confirmed (Fig. 2).

(ii)  Construction of plasmids pTB213 and pTB215

The plasmid pYJI [Goff, S. P., Cell, vide supra] comprising the A-MuLV provirus DNA cloned in the plasmid pBR322 at the HindIII site was cleaved with the restriction enzymes BamHI and PstI and a 1.2 kb DNA fragment containing the LTR region was isolated by agarose electrophoresis and purified.  Furthermore, the plasmid p8.2 [Niwa et al., vide supra] comprising the M-MuLV provirus DNA cloned in pBR322 at the HindIII site thereof was cleaved with the restriction enzymes PstI and ClaI and a 1.1 kb DNA fragment containing the LTR region was isolated and purified.

These LTR region-containing DNA fragments (each 1 μg) were respectively dissolved in 19 μl of T4 DNA polymerase buffer (33 mM Tris·acetate (pH 7.9), 66 mM potassium acetate, 10 mM magnesium acetate, 0.5 mM dithiothreitol, 100 μg/ml bovine serum albumin), and 1 μl of 2 mM dNTP (4 species; dATP, dCTP, dGT and dTTP) and 2.5 units of T4 DNA polymerase were added. The reaction was carried out at 37°C for 5 minutes and then terminated by adding 2.5 μl of 0.2 M EDTA (pH 7). DNA recovery was realized by extraction with phenol-chloroform (1:1) and precipitation with ethanol. To these DNA fragments thus rendered blunt at both ends, there was joined the HindIII linker (CAAGCTTG) by the method described in Example 1 (i).

Separately, the plasmid pTB106 constructed in Example 1 (i) was cleaved with the restriction enzyme HindIII (one relevant site occurring upstream from the SV40 promoter), and the phosphoryl group at the 5' end was eliminated by treatment with alkaline phosphatase.

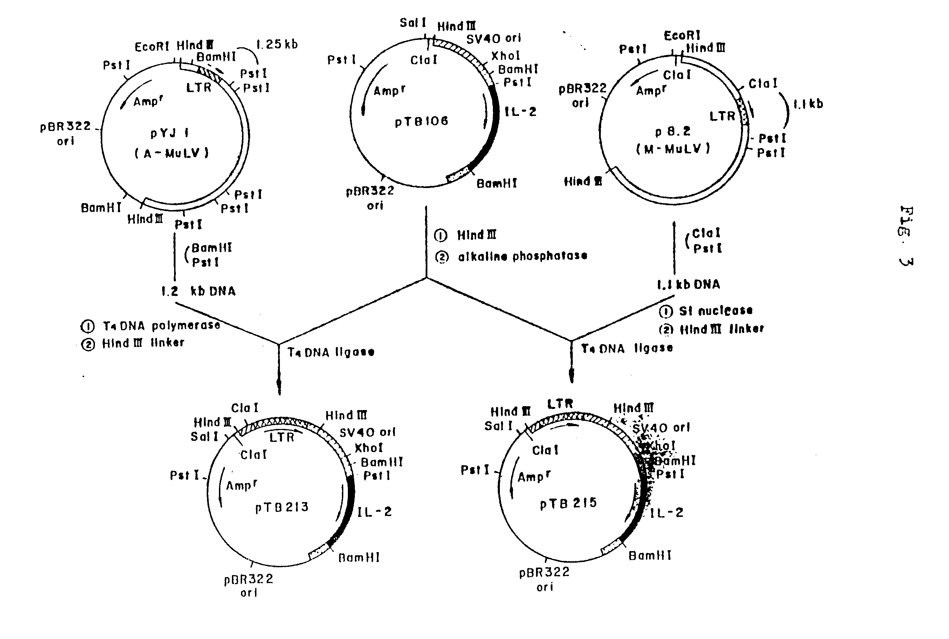

The HindIII linker-joined 1.2 kb A-MuLV LTR region DNA fragment or HindIII 1.1 kb M-MuLV LTR region DNA fragment was mixed with the pTB106 DNA HindIII fragment, and DNA ligation was effected under the action of T4 DNA ligase, whereby LTR region-containing plasmids for animal cell transformation, pTB213 (derived from A-MuLV) and pTB215 (derived from M-MuLV) were constructed (Fig. 3).

(iii) Construction of plasmid pTB314

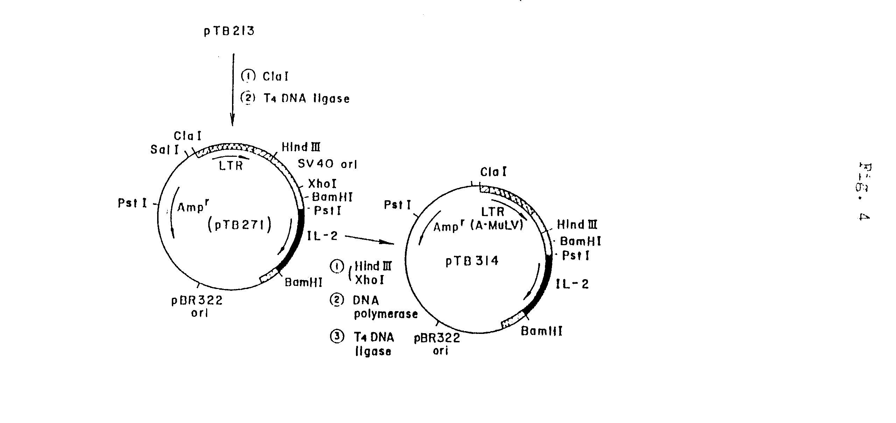

The plasmid pTB213 obtained in Example 1 (ii) was cleaved with the restriction enzyme ClaI and, after incubation at 65°C for 10 minutes, the ClaI was inactivated, followed by elimination, from pTB213, of a 0.13 kb ClaI DNA fragment upstream from the LTR region thereof by treating with T4 DNA ligase. Thus was constructed a recombinant, pTB271, with only one HindIII cleavage site occurring between the LTR region and the SV40 promoter region. This pTB271 was further cleaved with the restriction enzymes HindIII and XhoI, each cohesive end was rendered blunt by treating with T4 DNA polymerase in the presence of the four dNTPs, followed by ligation (without the SV40 promoter region) by the T4 DNA ligase reaction, whereby a plasmid for animal cell transformation, pTB314, having the A-MuLV LTR region as the promoter was constructed (Fig. 4).

(iv)   Construction of plasmid pTB385

pTB314 as obtained in Example 1 (iii) was cleaved with the restriction enzyme ClaI, rendered blunt-ended by    DNA polymerase reaction and joined with the HindIII linker (CAAGCTTG) by    T4 DNA ligase reaction. After cleavage with HindIII, a 3.3 kb DNA fragment was separated and purified by agarose electrophoresis and rendered circular by the T4 DNA ligase reaction, whereby a plasmid, pTB383, with the A-MuLV LTR region eliminated, was obtained. When an appropriate promoter is inserted into this plasmid at the SalI or HindIII cleavage site, the expression of IL-2 in aminal cells becomes possible.

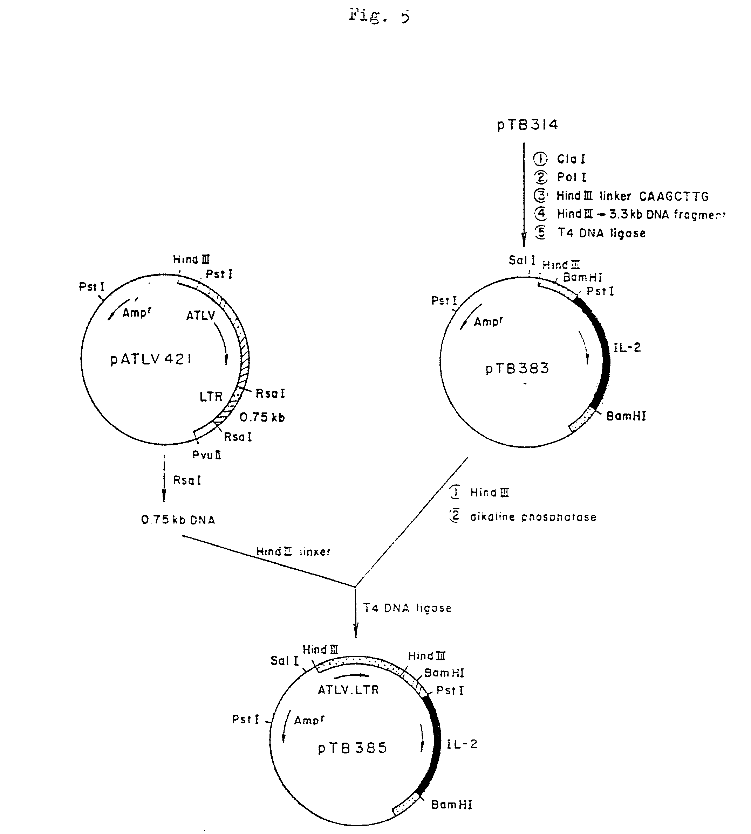

Separately, pATLV421 [plasmid cloned by Hatanaka et al. (Institute for Virus Research, Kyoto University) and having the nucleotides Nos. 5897-8894 of the ATLV genome (Yoshida et al., vide supra)] obtained by cloning the cDNA of ATLV (Yoshida et al., vide supra) by the method of Okayama and Berg [Okayama et al., Molecular and Cellular Biology, 2, 161-170 (1982)] was cleaved with RsaI and a 0.75 kb DNA fragment containing the ATLV LTR region was separated and purified, and joined with the HindIII linker (vide supra). This HindIII linker-joined 0.75 kb DNA fragment was mixed with HindIII-cleaved and alkaline phosphatase-treated pTB383 and the mixture was subjected to T4 DNA ligase reaction, whereby pTB385 was constructed (Fig. 5).

(v)  Construction of plasmids pTB485 and pTB487

Starting with pTB314 as obtained in Example 1 (iii), a plasmid, pTB399, was constructed by conversion of the HindIII cleavage site to a XhoI site and of the PstI cleavage site on the 5' end side of the IL-2 gene region to an EcoRI site and insertion of a BglII site directly before the BamHI cleavage site occurring at the 3' end of the IL-2 gene region and of ClaI and HindIII cleavage sites downstream from the poly(A) addition region.

Separately, from among a cDNA library prepared from hamster cell mRNA by the method of Okayama et al. [Molecular and Cellular Biology, 3, 280-289 (1983)], a DHFR gene cDNA-containing plasmid, pTB348, was selected. pTB348 had a 1.1 kb hamster DHFR cDNA and, when used for transfection of DHFR⁻ CHO cells, gave DHFR⁺ cells in a yield of about 100 colonies per microgram of DNA.

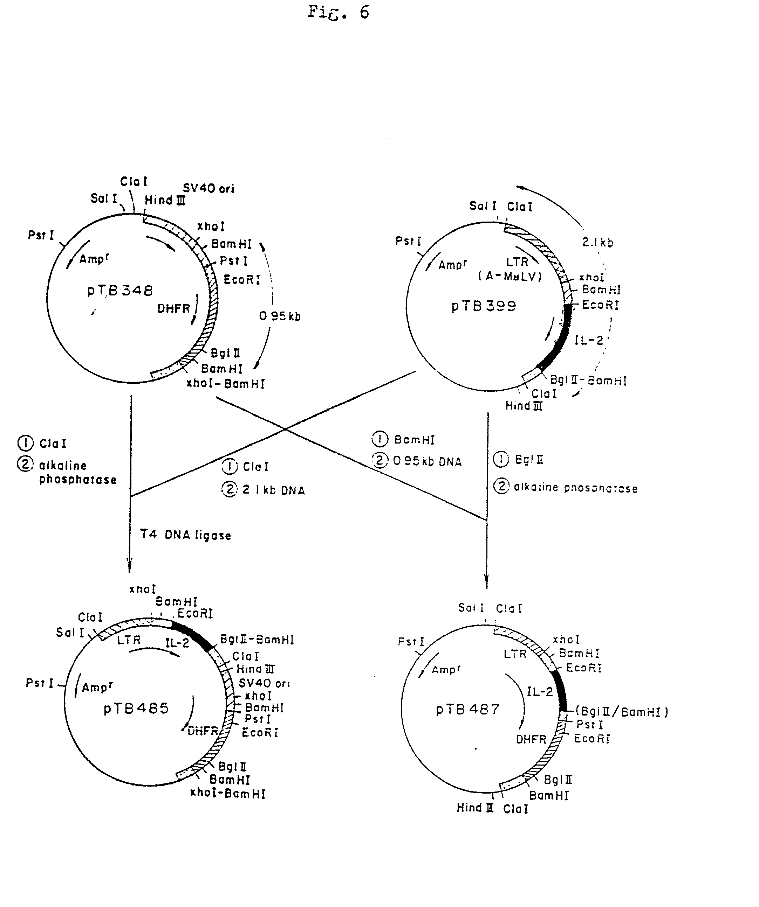

This plasmid, pTB348, was cleaved with ClaI, treated with alkaline phosphatase and mixed with a 2.1 kb DNA fragment of pTB399 as separated and purified following cleavage with ClaI [said fragment containing the A-MuLV LTR region, splicing region (SV40, 16S), IL-2 cDNA and poly(A) addition region (SV40, early)] and the mixture was subjected to T4 DNA ligase reaction, whereby a plasmid, pTB485, was constructed (Fig. 6).

pTB348 was cleaved with BamHI, a DHFR gene cDNA-containing 0.95 kb DNA fragment was separated and purified and then inserted into pTB399 at the BglII cleavage

- 32 -

017261g

site by the same method as mentioned above. Among from the plasmids obtained, the one with the DHFR gene inserted in the same direction as the IL-2 gene was selected and named pTB487 (Fig. 6).

pTB485 has a construction such that the IL-2 gene with A-MuLV LTR as the promoter therefor and the DHFR gene with the SV40 replication start region as the promoter therefor are connected in series in the same direction whereas pTB487 has only one promoter, namely A-MuLV LTR, and is a polycistronic vector, said promoter also controlling and allowing the expression of the DHFR gene occurring downstream from but close to the IL-2 gene.

(vi)  Transformation of animal cells

Eagle's MEM medium containing 10% of fetal bovine serum was placed in Falcon dishes (6 cm in diameter) and mouse TK-deficient L cells were cultured overnight at 37°C. Thereafter, these cells ($7 \times 10^5$ cells/dish) were blended for inoculation with 0.2 μg of the plasmid pTK61 (prepared by plasmid isolation from Escherichia coli LE578 [Gene, 7, 335-342 (1979); gift of Dr. Enquist] carrying a recombinant obtained by cloning a TK-gene-containing 3.5 kb BamHI DNA fragment of herpes simplex virus (HSV) in pBR322, and cloning a TK gene-containing 2 kb PvuII fragment [Proceedings of the National Academy of Sciences USA, 78, 1441-1445 (1981)] in pBR322) and 10 μg of the pTB106, pTB213, pTB215 or pTB314 DNA by the method of Graham et al.,
[Virology, 52, 456-467 (1973)]. After 4 hours of incubation
at 37°C, the medium was exchanged for a fresh portion and
the incubation was continued overnight. On the next day,
the medium was exchanged for HAT medium (MEM medium contain-
ing 15 µg/ml hypoxanthine, 1 µg/ml aminopterine, 5 µg/ml
thymidine and 0.25 µg/ml glycine) containing 10% of fetal
bovine serum, and incubation was continued at 37°C. After
about 2-3 weeks of incubation while making medium exchange
at 3- or 4-day intervals, the growth of $TK^+$ cells reached
the stage of colony formation.

Separately, the plasmid pTB106, pTB314 or pTB385
was introduced, together with the plasmid pTB6 [plasmid
derived from a plasmid containing the herpes simplex
virus-derived TK gene by substituting, for the TK structural gene region thereof, a 1 kb BclII-SmaI DNA frag-
ment containing the structural gene region of the neomycin resistance gene of the transposon Tn5; having the
same constitution as pAG60 of Colbert-Galapin et al.
[Journal of Molecular Biology, 150, 1-14 (1981)]], into
human FL cells under the same conditions as used for
TK-deficient L cells, with Eagle's MEM medium (containing
10% bovine fetal serum) supplemented with 800 µg/ml of
G418 ("Geneticin", Gibco) as the selective medium. Continued cultivation on the selective medium for 2-3 weeks
resulted in colony formation due to proliferation of
G418-resistant cells.

As for the plasmids pTB485 and pTB487, DHFR⁻ CHO cells [Urlaub et al., Proceedings of the National Academy of Sciences of USA, _77_, 4216-4220 (1980)] were cultivated on HAM's F12 medium containing 5% bovine fetal serum and transfected with 1 µg (for pTB485) or 5 µg (for pTB487) per dish of plasmid by the method of Graham et al. (vide supra). Two days later, the medium was replaced with Dulbecco's modified MEM medium containing 10% dialyzed bovine fetal serum and 35 µg/ml proline, followed by continued cultivation on this selective medium. After 2-3 weeks, the growth of cells which had become DHFR⁺ resulted in colony formation. The colony formation percentage for pTB487 was at most one tenth of that for pTB485.

(vii) Cloning of transformant cells

The transformant cells obtained in Example 1 (vi) were respectively cloned by a conventional method (e.g. limited dilution method). After cloning, the clones of L(TK⁺) cells and FL(G418ʳ) cells were cultivated on Eagle's MEM medium containing 10% bovine fetal serum and the clones of CHO(DHFR⁺) cells on Dulbecco's modified MEM medium containing 5% bovine fetal serum and 35 µg/ml proline. Cells of each clone isolated were scattered on a Linbro dish. When cell growth reached about 80% confluency, the medium was replaced with a fresh portion. After the subsequent 48 hours of incubation, the culture supernatant was assayed for IL-2 activity.

In the case of mouse L cells, among 9 transformant cell clones obtained with pTB106, clone 4 [L-IL106-4] showed the highest IL-2 activity of 0.6 U/ml. From among the transformant cells obtained with pTB213, there was isolated clone 3 [L-IL213-3] which showed an activity of 4.6 U/ml; from among the transformant cells obtained with pTB215, clone 4 [L-IL215-4], which showed an activity of 1.9 U/ml; and, from among the transformant cells obtained with pTB314, clone 12 [L-IL314-12], which showed an activity of 4.6 U/ml. The results are shown in Table 1.

Table 1

| Plasmid | Transformant (clone) | IL-2 activity U／ml |
|---------|----------------------|----------------------|
| pTB 106 | L-IL106-4 | 0. 6 |
| pTB 213 | L-IL213-2 | 2. 3 |
|         | L-IL213-3 | 4. 6 |
| pTB 215 | L-IL215-1 | 1. 3 |
|         | L-IL215-4 | 1. 9 |
| pTB 314 | L-IL314-9 | 3. 2 |
|         | L-IL314-12 | 4. 6 |

In the case of Human FL cells, the cells transformed with the plasmid pTB106 showed only an IL-2 activity as low as 0.2 U/ml or less. Similarly, the cells transformed with pTB314 showed an IL-2 activity as low as 0.2 U/ml [FL-IL314-1]. On the other hand, transformation with the ATLV promoter-containing plasmid pTB385 gave a clone showing an IL-2 activity of 1.5 U/ml [FL-IL385-6]. The results obtained are given in Table 2.

Table 2

| Plasmid | Transformant (clone) | IL-2 activity (U/ml) |
|---------|---------------------|----------------------|
| pTB106 | FL-IL106-1 | <0.2 |
| pTB314 | FL-IL314-1 | 0.2 |
| pTB385 | FL-IL385-3 | 0.7 |
| | FL-IL385-6 | 1.5 |

In the case of hamster CHO cells, transformation with the plasmid pTB485 gave a clone showing an IL-2 activity of 2.0 U/ml [clone C-IL485-4] and a clone showing an IL-2 activity of 0.3 U/ml [C-IL485-5]. On the other hand, transformation with the plasmid pTB487, which can be considered to be a polycistronic vector, gave a clone showing an IL-2 activity of 10.5 U/ml [C-IL487-10]. The results obtained are shown in Table 3.

(viii)   Gene amplification

The IL-2-producing CHO cell strain C-IL485-4 obtained in Example 1 (vii) was grown on Dulbecco's modified MEM medium (containing 5% bovine fetal serum and 35 μg/ml proline) with 10 nM methotrexate (MTX) added. This clone showed normal growth in the presence of MTX in said concentration. Therefore, the MTX concentration was increased to 100 nM and subculturing was continued. When the MTX concentration was raised to 1 μM, most cells died. However, continued incubation with medium exchange at 3- or 4-day intervals resulted in the start of growth, in the form of colonies, of several cells out of $10^5$ cells. After sufficient growth of these cells, the cells were subcultured on a medium having an MTX concentration of 10 μM, whereupon, again, most of the cells died, a few cells  alone  showing colony-forming proliferation. The cells thus obtained showed stable and normal growth in the presence of 10 μM MTX and, when returned to an MTX-free medium and then, after several subcultures, cultivated in the presence of 10 μM MTX, grew normally. The C-IL485-4 cells resistant to 10 μM MTX produced 59.0 U/ml of IL-2 in the culture medium under the same conditions as used in Example 1 (vii), the yield being about 30 times as compared with the starting clone.

- 38 -

0172619

Also with the cell strains C-IL485-5 and C-IL487-10, cells resistant to 10 µM MTX were isolated by the same method. In either case, MTX-resistant cells gave an increased IL-2 activity in the culture medium as compared with the starting cells. The results obtained are shown in Table 3.

Table 3

| Plasmid | Transformant (clone) | IL-2 activity (U/ml) | |
|---------|---------------------|---------------------|---|
| | | Starting cells | MTX (10 µM)-resistant cells |
| pTB485 | C-IL485-4 | 2.0 | 59.0 |
| pTB485 | C-IL485-5 | 0.3 | 4.1 |
| pTB487 | C-IL487-10 | 10.5 | 20.3 |

Example 2   Cultivation of transformant

With the animal cell transformant L-IL314-12 obtained in Example 1 (vii), the culture supernatant IL-2 activity was assayed at timed intervals. Thus, Falcon dishes, 3.5 cm in diameter, were each seeded with 1.5 x 10$^5$ L-IL314-12 cells and cultivation was conducted in 2 ml of MEM medium containing 10% of fetal bovine serum at 37°C in a $CO_2$ incubator. Starting from 1 day after commencement of incubation, the number of cells and the culture supernatant IL-2 activity were followed daily (Fig. 7). IL-2 was produced and accumulated as the cell proliferation proceeded and, even after the cell proliferation had subsided, the production of IL-2 continued. The maximum activity attained was 24.6 U/ml.

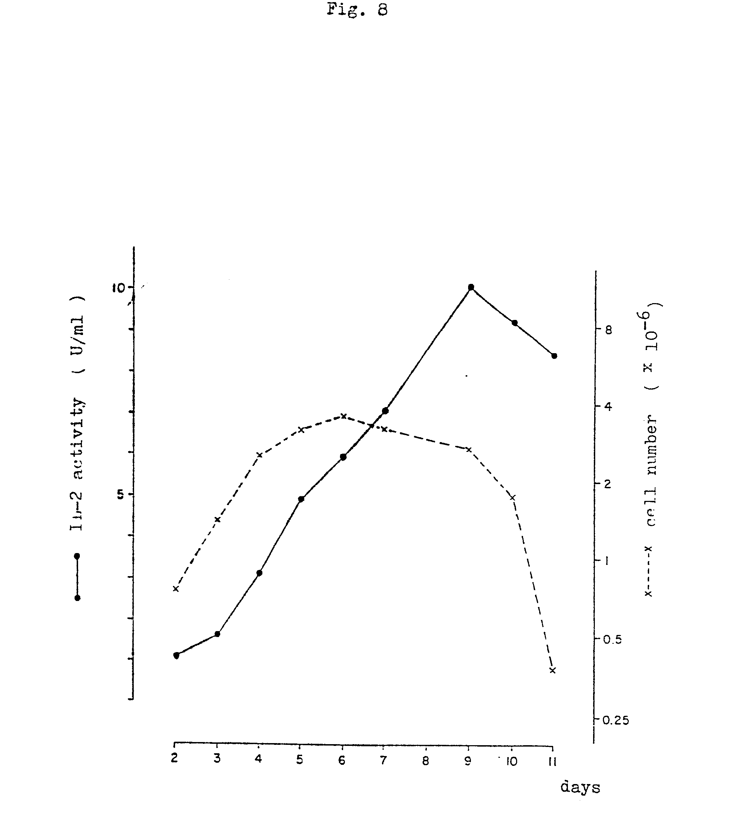

Also for the FL-IL385-6 cells obtained by introduction of the plasmid pTB385 into human FL cells, the IL-2 activity in the culture medium was assayed at timed intervals. Onto Eagle's MEM medium containing 10% bovine fetal serum in a Falcon dish (3.5 cm in diameter), there were dispersed $3 \times 10^5$ cells. On the next day, the medium was replaced with a fresh 2-ml portion of the same medium. Thereafter, the cell count and IL-2 activity in the culture medium were determined every day [Fig. 8]. Like the case of L-IL314-12 cells, IL-2 was produced and accumulated with the growth of cells. The IL-2 production continued even after termination of cell growth and attained a maximum activity of 10.1 U/ml.

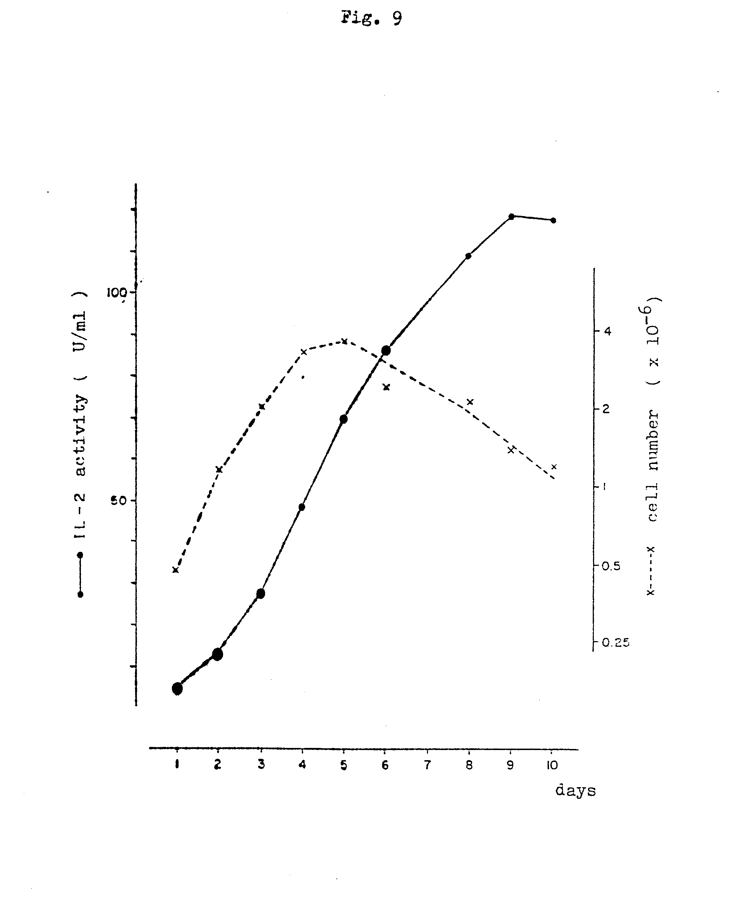

The IL-2 production was determined at timed intervals also for the transformant cell strain C-IL485-4 and the clone C-IL485-14 from MTX (10 μM)-resistant cells of said strain. The culture medium used was Dulbecco's modified MEM medium containing 5% bovine fetal serum and 35 μg/ml proline and other conditions were the same as above [Fig. 9]. The maximum IL-2 activity was 119 U/ml.

Example 3  Separation of glycosylated human IL-2

The animal cell transformant (clone) L-IL213-3 obtained in Example 1(vii) was grown in MEM medium containing 10% of fetal bovine serum.  Cells were cultured on Falcon dishes (Falcon), 3 cm in diameter, and, on day 2, the medium was exchanged for a methionine-free MEM medium containing $^{35}$S-methionine in an amount of 50 µCi ($10^3$ Ci/mM).  Continued cultivation resulted in labeling of the IL-2 molecule secreted out of cells.  After continued cultivation in MEM medium containing $^{35}$S-methionine, the culture broth and cells were collected within 1-72 hours and centrifuged at 15,000 rpm and 4°C for 20 minutes.  To a 180-µl portion of the supernatant thus obtained, there was added 20 µl of anti-human IL-2 antiserum (rabbit) and the mixture was allowed to stand at 37°C for 1 hour and then at 4°C for 24 hours.  Then, 100 µl of 10% protein A suspension (0.05 M Tris-0.15 M NaCl, 5 mM EDTA pH 7.4, 0.05% NP-40) was added and the whole mixture was allowed to stand at 4°C for a further 1-2 hours [Journal of Immunology, 115, 1617-1624 (1975)].  The protein A-bound antigen-antibody complex was collected by centrifugation at 12,000 rpm for 2 minutes, the precipitate was washed 4 or 5 times with NET buffer containing 0.05% of NP-40 and then suspended in 30 µl of a buffer for polyacrylamide gel electrophoresis samples, and the suspension was heated at 100°C for 5 minutes. After centrifugation, the supernatant collected was subjected to 17% polyacrylamide gel electrophoresis (40 volts, 17 hours) [Nature, 227, 680-685 (1971)]. The gel was then fixed with 50% trichloroacetic acid and subjected to fluorography. On the autoradiogram obtained, the IL-2 molecule reacting with anti-IL-2 antiserum was detected as two bands about 14 kilodaltons and 16-17 kilodaltons in molecular weight (Fig.10).

CLAIMS

1. An animal cell transformed with a DNA having a region coding for a human interleukin-2 protein and at least one promoter upstream therefrom.

2. The transformant according to claim 1, wherein the DNA further has at least one enhancer.

3. The transformant according to claim 2, wherein the enhancer is an animal virus-derived enhancer.

4. The transformant according to claim 1, wherein the promoter is an animal virus-derived promoter.

5. The transformant according to claim 3 or 4, wherein the animal virus is simian virus.

6. The transformant according to claim 3 or 4, wherein the animal virus is murine retrovirus.

7. The transformant according to claim 3 or 4, wherein the animal virus is human retrovirus.

8. The transformant according to claim 2, wherein the enhancer is an enhancer of the repeating base sequence portion in a retrovirus LTR region.

9. The transformant according to claim 2, wherein the enhancer is an enhancer of simian virus 40 promoter region.

10. The transformant according to claim 2, wherein the enhancers are those of the repeating base sequence portion in a retrovirus LTR region and of simian virus 40 promoter region.

11. The transformant according to claim 1, wherein the promoter is a promoter derived from a retrovirus LTR region.

12. The transformant according to claim 1, wherein the promoter is a promoter derived from simian virus 40 promoter region.

13. The transformant according to claim 1, wherein the promoters are those derived from a retrovirus LTR region and derived from simian virus 40 promoter region.

14. The transformant according to claim 1, which further carries a gene amplification gene.

15. The transformant according to claim 14, wherein the gene amplification gene is dihydrofolate reductase gene.

16. The transformant according to claim 1, wherein the animal cell is a mouse cell.

17. The transformant according to claim 1, wherein the animal cell is a human cell.

18. The transformant according to claim 1, wherein the animal cell is a hamster cell.

19. The transformant according to claim 1, wherein the transformation has been carried out by co-transformation.

20. The transformant according to claim 1, which is Mouse L-IL213-3 cell.

21. The transformant according to claim 1, which is Human FL-IL385-6 cell.

22. The transformant according to claim 1, which is Hamster C-IL485-14 cell.

23. A method of producing a human interleukin-2 protein which comprises cultivating the transformant defined in claim 1 to thereby cause production and accumulation of said protein in the culture and recovering the same.

24. The method according to claim 23, wherein the human interleukin-2 protein is glycosylated human interleukin protein.

25. The method according to claim 23, wherein the cultivation is carried out under the gene amplification codition.

Fig. 1

5'GGGGGGGGGGGGGGGGGGATCACTCTCTTTAATCACTACTCACAGTAACC

S1
TCAACTCCTGCCACA ATG TAC AGG ATG CAA CTC CTG TCT TGC

                                            S20  1
ATT GCA CTA AGT CTT GCA CTT GTC ACA AAC AGT GCA CCT

ACT TCA AGT TCT ACA AAG AAA ACA CAG CTA CAA CTG GAG

              20
CAT TTA CTG CTG GAT TTA CAG ATG ATT TTG AAT GGA ATT

                                        40
AAT AAT TAC AAG AAT CCC AAA CTC ACC AGG ATG CTC ACA

TTT AAG TTT TAC ATG CCC AAG AAG GCC ACA GAA CTG AAA

          60
CAT CTT CAG TGT CTA GAA GAA GAA CTC AAA CCT CTG GAG

                                            80
GAA GTG CTA AAT TTA GCT CAA AGC AAA AAC TTT CAC TTA

AGA CCC AGG GAC TTA ATC AGC AAT ATC AAC GTA ATA GTT

              100
CTG GAA CTA AAG GGA TCT GAA ACA ACA TTC ATG TGT GAA

TAT GCT GAT GAG ACA GCA ACC ATT GTA GAA TTT CTG AAC

120
AGA TGG ATT ACC TTT TGT CAA AGC ATC ATC TCA ACA CTG

133
ACT TGA TAATTAAGTGCTTCCCACTTAAAACATATCAGGCCTTCTATTT

ATTTAAATATTTAAATTTTACCCCCCCCCCCCCCC3'

Fig. 2

3 / 10

Fig. 3

Fig. 4

4 / 10

Fig. 5

Fig. 7

Fig. 8

Fig. 9

Fig. 10

European Patent
Office

**EUROPEAN SEARCH REPORT**

**0172619**
Application number

| | **DOCUMENTS CONSIDERED TO BE RELEVANT** | | EP 85304383.4 |

EP 85304383.4

| Category | Citation of document with indication, where appropriate, of relevant passages | Relevant to claim | CLASSIFICATION OF THE APPLICATION (Int Cl 4) |
|---|---|---|---|
| X | EP - A1 - 0 091 539 (AJINOMOTO CO., INC.; JAPANESE FOUNDATION FOR CANCER RESEARCH)<br><br>* Claims 19,24,29 * | 1,23 | C 12 N 15/00<br>C 12 N 5/00<br>C 12 N 7/00<br>C 12 P 21/02<br>C 07 K 3/00<br>C 07 K 13/00 |
| D,A | MOLECULAR AND CELLULAR BIOLOGY, vol. 3, no. 1, January 1983 (Washington D.C.)<br><br>H. OKAYAMA et al. "A cDNA Cloning Vector that Permits Expression of cDNA Inserts in Mammalian Cells" pages 280-289<br><br>* Page 280 * | 1,5 | |
| D,A | CELL, vol. 22, no. 3, December 1980 (Cambridge, Mass.)<br><br>S.P. GOFF et al. "Structure of the Abelson Murine Leukemia Virus Genome and the Homologous Cellular Gene: Studies with Cloned Viral DNA" pages 777-785<br><br>* Summary * | 1,6 | **TECHNICAL FIELDS SEARCHED (Int Cl 4)**<br><br>C 12 N<br>C 12 P<br>C 07 K |
| D,A | PROCEEDING OF THE NATIONAL ACADEMY OF SCIENCES OF THE UNITED STATES OF AMERICA, vol. 79, no. 22, November 1982 (Baltimore, USA)<br><br>M. SEIKI et al. "Human adult T-cell leukemia virus: Molecular cloning of the provirus DNA and the unique terminal structure" pages 6899-6902<br><br>* Abstract * | 1,7 | |

The present search report has been drawn up for all claims

| Place of search | Date of completion of the search | Examiner |
|---|---|---|
| VIENNA | 24-09-1985 | WOLF |